Europäisches Patentamt

European Patent Office

Office européen des brevets

(11) Publication number: **0 182 539**
**B1**

(12) **EUROPEAN PATENT SPECIFICATION**

(45) Date of publication of patent specification: **04.04.90**

(51) Int. Cl.⁵: **A 61 M 25/02**

(21) Application number: **85308048.9**

(22) Date of filing: **05.11.85**

(54) **Gastro-jejunal feeding device.**

(30) Priority: **05.11.84 US 670381**
**22.10.85 US 790242**

(43) Date of publication of application:
**28.05.86 Bulletin 86/22**

(45) Publication of the grant of the patent:
**04.04.90 Bulletin 90/14**

(84) Designated Contracting States:
**AT BE CH DE FR GB IT LI LU NL SE**

(56) References cited:
**DE-A-1 616 493**
**FR-A-2 499 858**
**FR-A-2 551 978**
**US-A-2 457 244**

**US-A-3 241 554**
**US-A-3 487 837**
**US-A-4 543 100**

**Journal of Parenteral and Enteral Nutrition, 8, 2,
1984, pages 203-207**

**The file contains technical information
submitted after the application was filed and
not included in this specification**

(73) Proprietor: **MEDICAL INNOVATIONS
CORPORATION**
**1595 McCandless Drivee**
**Milpitas California 95035 (US)**

(72) Inventor: **Parks, Stephen K.**
**1283-216 Vicente Drive, Sunnyvale**
**California 94086 (US)**

(74) Representative: **Beresford, Keith Denis Lewis
et al**
**BERESFORD & Co. 2-5 Warwick Court High
Holborn**
**London WC1R 5DJ (GB)**

Courier Press, Leamington Spa, England.

## Description

This device relates to a catheter device for the alimentary canal of the human or animal body, such as gastro-jejunal feeding tube that is insertable through a stoma in the patient's stomach wall and secured within the patient's stomach and against the abdominal wall. Alternatively, the device may be employed to by-pass the stomach and feed directly into the jejunum, or for drainage and/or decompression.

Some types of patient feeding devices employ a gastrostomy feeding tube. However, if the patient has a problem with gastric reflux or vomiting, or if the stomach is not adequate for the patient's digestive process requirements, another feeding mode must be chosen. This can be accomplished by by-passing the stomach and supplying food directly to the jejunum with a feeding tube.

Many types of feeding devices have been developed, but they suffer from various drawbacks. These include: the ejection or loss of liquids from the stomach and back through the device; leaking around the periphery of the device, and, premature deterioration of the materials of construction. Also, it is difficult to maintain the device in place in a stable manner in the patient, and this latter problem can result in the device being ingested into the stomach, and eventually into the pylorus. Devices presently on the market are not sized properly, and they use materials that are prone to fairly rapid deterioration.

Moreover, they can become entangled and dislodged from the patient due to improper sizing and inadequate locking of the device to the patient. In some prior art devices, the exterior of the gastrostomy tube is taped to the wearer's body. This procedure can cause infection at the stoma entry, and along the taped area, as well as causing irritation due to the difficulty in maintaining these areas clean.

US—A—4543100, which is published after the priority date of the present application, discloses a balloon type catheter device in which an external retaining member is used. The retaining member is in the form of a ring which is attached to the skin by means of adhesive and which has a membrane through which the catheter tube passes. In Journal of Parenteral and Enteral Nutrition, 8, 2, 1984 pp 203—207, G. Moss describes a catheter device according to the prior-art portion of claim 1 and discusses the use of a gastromy catheter which has "a snug adjustable silicone collar on the external segmen of the (tube) positioned to maintain minimal traction and protect against inward migration." In practice, this type of collar was attached to the tube by an adhesive tape and attached to the patient's skin by sutures to hold it in place.

Other prior art devices employ a spring biased or threaded locking mechanism to secure a locking ring to the wearer's body, the locking ring being fastened on the gastrostomy tube. But all these devices maintain a fixed pressure or position of the locking ring on the gastrostomy tube, and do not self adjust to peristaltic pressure of the stomach. This is of particular importance in the case of neonatal patients or others such as incoherent or unconscious patients who are unable to communicate the nature of their discomfort. Generally, the use of tape or mechanical locking devices requires extra care which is usually provided by trained personnel such as nurses.

The invention provides a catheter device for the alimentary canal of the human or animal body comprising:

a tube to project through a stoma into the alimentary canal;

a closure to block the tube;

a first abutment provided on the tube to abut the wall of the alimentary canal, and

a second abutment slidable along the tube to abut the external surface of the body around the stoma,

the tube and the abutment being suitable for prolonged implantation in the body, characterised in that the frictional contact between the tube and the second abutment is (a) sufficiently high to prevent the tube from being drawn through the second abutment into the stomach by peristalsis, but (b) sufficiently low to permit outward movement of the second abutment if the peristaltic pressure of the alimentary canal becomes too great.

According to one preferred embodiment of the invention, a gastrostomy or jejunostomy feeding tube is provided for insertion through the stoma of the patient's stomach wall and into the patient's stomach and/or jejunum. The feeding tube is provided with an inflatable balloon at one end to position and secure the tube within the stomach. The outer end of the tube is provided with a moveable locking ring that can be adjusted by frictional engagement of the locking ring along the gastrostomy or jejunal feeding tube to accommodate to the size of the wearer. Since the locking ring does not require or employ tape to secure the device in place, problems associated with skin irritation and with maintaining both the taped areas and the stoma area clean are greatly reduced. The locking ring can simply be moved along the tube against the frictional force between the ring and the tube to permit cleaning of the stoma entry through which the catheter is inserted. The locking ring is then repositioned to its normal location, i.e. in close contact with the wearer's abdomen.

The balloon and locking ring thus both function to maintain the device in place, and prevent the device, by frictional engagement between the locking ring and tube, from being drawn into the stomach, or being inadvertently pulled out. If desired, a mushroom tip can be used in place of a balloon, and this can be important during the first few post-operative days when a balloon tip is more vulnerable to being ruptured.

In the case of a gastro-jejunal feeding system, one embodiment of the invention involves extending the tube through the stomach and feeding directly into the jejunum. In another embodiment, the feeding system of this invention permits liquid food to be supplied to the jejunum while the stomach is drained by means of a comounted gastrostomy tube. This represents a significant benefit for patients who have a problem with gastric reflux or vomiting. However, if the stomach function is only somewhat impaired and vomiting or gastric reflux are not a problem with a particular patient, liquid food can be supplied to both the jejunum and the stomach through both tubes.

In another embodiment, the device may be adapted to feed directly through a jejunal stoma and into the jejunum.

The device of this invention is preferably constructed of a medical grade silicone elastomer rather than a latex or silicone latex combination. Consequently, use of the silicone elastomer provides a suitably inert material compared to the latex. Hence, the elastomer requires replacement about every 6—8 weeks compared to a silicone latex which needs replacing about every three weeks.

There follows a description by way of example of specific embodiments of the invention, reference being made to the accompanying drawings, in which:

Fig. 1 is an external perspective view of the gastrostomy device of this invention;

Fig. 2 is an external perspective view of the said device partly fragmented, showing the outlet end, and the balloon when deflated;

Fig. 3 is a perspective view, partly broken away, of the said device installed in a patient;

Fig. 4 is an external perspective view of a preferred form of locking ring employed in the device;

Fig. 5A is an external perspective view showing the use of a mushroom tip in place of the balloon, together with a perforated and ribbed locking ring;

Fig. 5B is an external, perspective view of the said device for feeding into the jejunum via a stomach placed stoma;

Fig. 6 is an external, perspective view of the said device for feeding into both the stomach and jejunum via a stomach placed stoma; and

Fig. 7 is an external, perspective view of the said device adapted for by-passing the stomach and feeding directly into the jejunum.

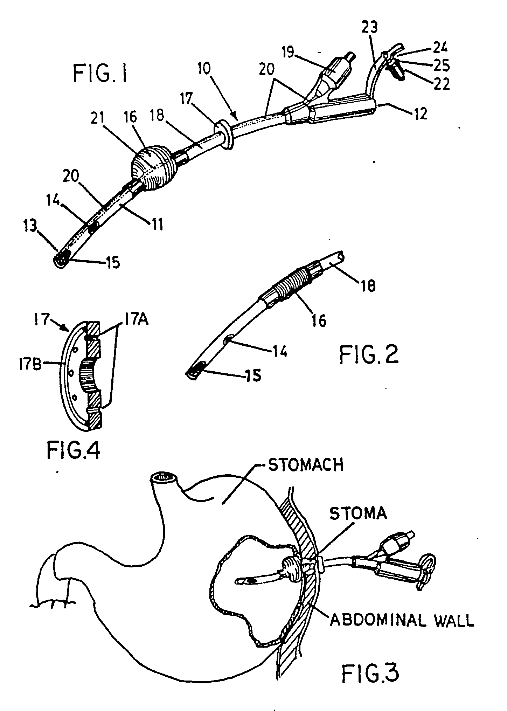

The gastrostomy catheter device 10 of this invention is shown in Fig. 1, and provides an inlet end 12, through which is fed food and medication, and an outlet end 13 that extends into the patient's stomach. A plurality of outlet ports, two of which are shown, are located at the outlet end 13. The catheter 10 is secured inside the stomach by an inflatable balloon 16, and on the patient's abdomen by an adjustable silicone locking ring 17. As shown in Fig. 4, the locking ring 17 is provided with a plurality of vent holes 17A and a circular ridge 17B to permit air to contact the entry to the stoma and reduce infection and skin irritation.

As indicated, use of the ring prevents the catheter from being drawn into the patient's stomach. In addition, since the adjustable ring does not require the use of tape, a potential source of skin irritation and infection is eliminated. The portion 18 of the catheter tube 11 between the ring 17 and balloon 16 is secured within the stoma, and this arrangement of the balloon and ring prevents the catheter from being drawn into the patient's stomach.

In Fig. 1, the balloon is inflated by liquid or gas which is passed through a valve 19 and line 20 into port 21 that is surrounded by the balloon. The line 20 is bonded along the inside of the catheter tube 11 and extends to the outlet of the catheter where it is end sealed; the end seal forces the inflating gas into the port 21. Fig. 2 shows the balloon 16 is a deflated position.

The inlet end 12 is provided with an integrally formed end plug 22 attached to the catheter by a band 23. A plurality of rings, 24, 25 are formed on the plug to engage corresponding grooves (not shown) on the inside of the bore at the inlet. The combined effect of the plug and bore fit, and the fit between the grooves and rings prevent the plug from being dislodged during use, and hence, will prevent the contents of the stomach from draining out through the catheter.

Basically, the catheter device is inserted into the patient through a surgically prepared stoma created in the abdominal wall using pre-existing surgical procedures. These procedures include Stamms Gastrostomy, Witzel Gastrostomy, and others. Also, non surgical procedures may be employed such as percutaneous gastrostomy. The Janeway surgical procedure may also be used.

The catheter tube 10, with surrounding, concentric purse string sutures, is inserted through the stoma and gastric wall into the stomach. The purse strings will permanently invaginate a portion of the stomach and stoma to shape around the catheter tube and then will dissolve, leaving the gastrostomy tube in place and ready for use. Fig. 3 shows the device when installed. The inflated balloon forms a gasket that seals the entrance to the stoma, and along the locking ring 17, secures the device in place. The device may be constructed in various sizes to accommodate a particular patient. Sizes such as 12, 14, 16, 18 and 20 French, and corresponding diameters varying from about 0.157"—0.263", (4—6.7 mm), and a wall thickness of about 0.035" (0.9 mm), may be used.

After being used for a suitable time, say 6 to 8 or 9 weeks, the catheter tube is, of course, replaced. This is accomplished simply by deflating the balloon, retracting the adjustable ring, and removing the tube from the patient.

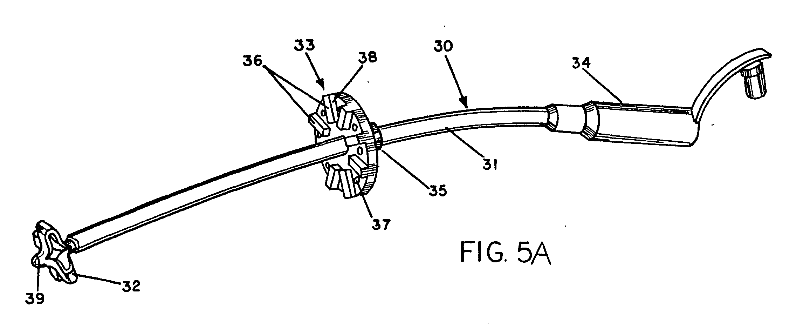

Fig. 5A illustrates the use of a mushrom tip employed in place of a balloon, and a locking ring designed to provide air circulation between the ring and stoma areas to reduce irritation and the possibility of infection. The gastrostomy catheter device 30 includes a tube portion 31, mushroom tip 32, adjustable locking ring 33, and food inlet portion 34. The locking ring 33 includes a collar 35 and a plurality of raised, concentric legs 36, air vents 37, and air gaps 38 between the legs 36 to permit circulation of air between the ring and stoma area. The mushroom tip 32 is inserted (and removed) with a stylet through the tube and expands to its final shape in the stomach when it is pushed through the tube. The food inlet 34 has the same construction as that shown in Fig. 1.

It will be appreciated that while a balloon or mushroom tip are described above for securing the device internally, components such as a plate may be used as a substitute for the balloon or mushroom tip components.

The operation of the locking ring 33 is the same as that of the ring 17 in Fig. 1. Basically, the locking ring 33 is adjustable along the tube 31 and forms a light friction lock against the user's abdomen. When the stoma area requires cleaning, the ring 33 is simply retracted against the frictional force between the ring and tube 31, and then forced back against the abdomen after the stoma area has been cleaned. Usually, the locking ring 33 and tube 31 are both made of medical grade silicone material.

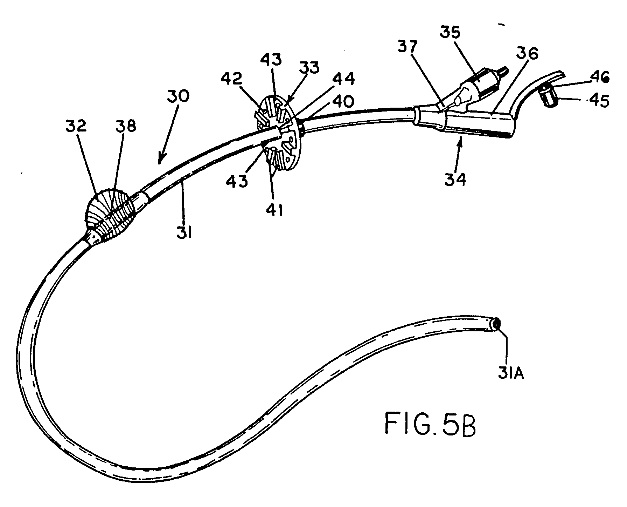

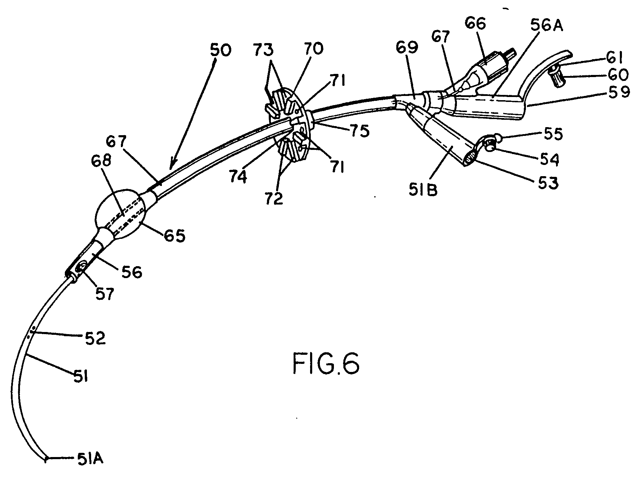

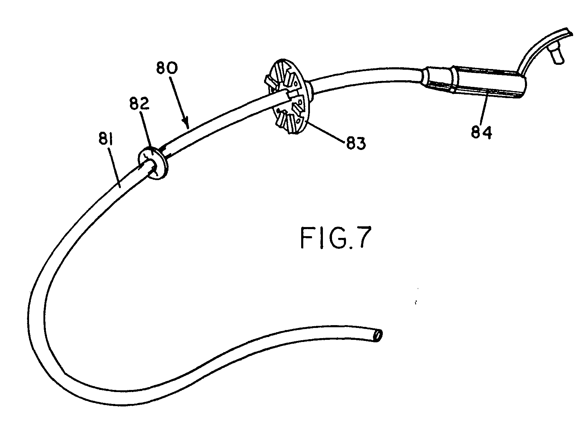

Figs. 5B and 6 illustrate the use of a gastrostomy placed stoma for feeding into the jejunum (Fig. 5B), or into the stomach and/or the jejunum (Fig. 6). Fig. 7 illustrates the use of a jejunum placed stoma which by-passes the stomach and feeds directly into the jejunum.

In Fig. 5B, the jejunum feeding device 30 includes a feeding tube 31 having an open end 31a that extends through the stomach and into the jejunum. The device provides an expandable balloon portion 32, locking ring 33 and an end member 34 having an inflating valve 35 and feed inlet 36. A gas line 37 is bonded along the interior of the tube and joins the interior of the balloon with the valve. An outlet port 38 enables the balloon to be filled from the valve via the gas line.

The locking ring 33 includes a collar 40 and plurality of raised, concentric ribs 41, air vents 42, and air gaps 43 between the legs to permit circulation of air between the ring and stoma area. The central portion of the locking ring defines a bore 44 through which passes the feeding tube 31. The frictional contact between the bore wall and collar 40 with the tube is sufficiently great to permit the locking ring to remain in contact with the patient's abdominal wall during use. However, the frictional contact between the tube and locking ring is sufficiently low so as to permit outward movement of the locking ring from the patient if the peristaltic pressure of the stomach becomes too great. Also, the frictional contact between the locking ring 33 and the tube is sufficiently low to permit the locking ring to be moved away from the abdominal wall and enable the stoma area to be cleaned.

As in Fig. 1, the feed inlet 36 includes an attached cover plug 45 which can be inserted into the inlet and remain in place by means of a ring 46 that engages a corresponding groove (not shown) on the inside of the bore at the inlet.

The jejunal feeding tube 30 is inserted into the patient in the same manner as for the gastrostomy tube shown in Fig. 1. Since the jejunal tube is about 2 feet (600 mm) longer than the gastrostomy tube, it can be fed directly into the jejunum through a simple gastrostomy stoma.

The embodiments shown in Figs 5B and 6 represents a considerable advantage to a patient who has a problem with gastric reflux or vomiting, but does not require gastric decompression or drainage. In Fig. 6, the jejunal feeding tube device 50 includes a jujunal feeding tube 51, having an outlet end portion 51a which extends through the stomach into the jejunum, and which is perforated at 52 to permit liquid food to be passed therethrough. The tube 51 connects at its inlet end 51b to a food inlet port 53 having a connecting plug cover 54 bearing one or more circular ridges 55. The plug cover can be inserted into the inlet port and will be secured therein by corresponding grooves (not shown) that engage the ridges 55.

Surrounding the feeding tube 51 is a shorter length gastrostomy tube 56 having a plurality of drainage inlets or food outlet ports, one such port 57 being shown. The drainage outlet (of food inlet) end 56a is enlarged to form a port 59 having an attached cover plug 60. One or more circular ridges 61 formed on the plug engages corresponding grooves (not shown) on the inside of the entry port to ensure a tight fit when the entry port 59 is closed.

An inflatable balloon 65 is provided near the end of the gastrostomy tube and is inflatable through a valve 66 bonded into the drainage (or inlet) end 56a. The valve is used to supply air to the balloon through a connecting line 67 and outlet port 68 under the balloon.

A connector 69 is employed to join the jejunum tube 51, inlet end 51b, gastrostomy tube 56, and drainage (or feeding) end 56a into a rigid package.

An adjustable, silicone ring 70 is provided with a plurality of vent holes 71 to permit air to contact the entry to the stoma and reduce infection and skin irritation. This is aided by a series of concentric ribs 72 being spaced at 73 for circulation of air therebetween. The central portion of the locking ring defines a bore 74 through which pass the jejunal and gastrostomy tubes 51 and 56. Frictional contact between the bore 74 and collar 75 with the gastrostomy tube 56 is sufficiently great to permit the locking ring to remain in contact with the patient's abdominal wall during use. However, the frictional contact between the tube and locking ring is sufficiently low so as to permit putward movement of the locking ring from the patient if the peristaltic pressure of the stomach becomes too great. Also the frictional contact between the locking ring 70 is sufficiently low to enable the locking ring to be retracted from the abdominal wall and permit the stoma area to be cleaned. As in Fig. 5B. the already existing stoma can serve either as an entry for a jejunal tube or for a gastrostomy tube without any additional surgery being required.

In Fig. 7, use of an inflatable balloon is eliminated, and a plate is employed instead, The jejunum feeding device 80 is shown providing a jejunal tube 81, retaining plate 82, locking ring 83 and food inlet 84. The device is secured permanently by means of the plate 82 constructed of say a silicone material, which presses against the entrance to the stoma on the inside of the jejunal wall, and by the locking ring 83 which is pressed against the wearer's abdomen at the stoma entry. As in the other embodiments, the extent of frictional engagement between the locking ring 83 and the tube 81 is sufficient to prevent the device from being drawn into the user by peristalsis, while still enabling the ring to move outwardly due to expansion, or to be retracted so that the stoma may be cleaned.

The present device is inexpensive and can be readily manufactured by conventional extrusion and injection molding techniques. Also, it can be easily inserted for use without generally requiring the services of a physician or even outpatient services. Furthermore, when in hospital, nursing time with its attendant costs have been found to be reduced significantly.

The device may be cleaned during use and can be manipulated to permit cleaning of the stoma area. Finally, the device is safe in that it cannot be drawn into the user, which can be particularly dangerous to unsuspecting infants and incoherent or unconscious patients, or the like. During use, it will not inadvertently drain the contents of the stomach or jejunum because of the end plug.

## Claims

1. A catheter device for the alimentary canal of the human or animal body comprising:

a tube (11, 31, 51, 81) to protect through a stoma into the alimentary canal;

a closure (22, 45, 60) to block the tube;

a first abutment (16, 32, 65, 82) provided on the tube to abut the wall of the alimantary canal, and

a second abutment (17, 33, 40, 70, 83) slidable along the tube to abut the external surface of the body around the stoma.

the tube and the abutment being suitable for prolonged implantation in the body,

characterised in that the frictional contact between the tube (11, 31, 51, 81) and the second abutment (17, 33, 40, 70, 83) is (a) sufficiently high to prevent the tube (11, 31, 51, 81) from being drawn through the second abutment (18, 33, 40, 70, 83) into the stomach by peristalsis, but (b) sufficiently low to permit outward movement of the second abutment (17, 33, 40, 70, 83) if the peristaltic pressure of the alimentary canal becomes too great.

2. A device according to claim 1, characterised in that the tube and the second abutment are formed of a medical grade silicone elastomer.

3. A device according to claim 1 or 2, wherein the second abutment is provided with a plurality of vent holes (17a) to permit air to contact the external surface of the body.

4. A device according to claim 3, wherein the second abutment is provided with ridges or ribs (17b, 72) for permitting air to circulate between the second abutment and the external surface of the body.

5. A device according to any preceding claim, characterised in that the tube is a feeding tube (11) having a feeding inlet end (12) and a perforated outlet end (13) for discharging food, and in that the closure plug (22) for the feeding inlet is secured to the feeding tube by an integral band (23).

6. A device according to any preceding claim, characterised in that the second abutment is a locking ring (17, 33, 70, 83) through which the tube is a sliding fit.

7. A device according to any preceding claim, characterised in that the tube comprises a jejunal feeding tube (51) and a gastrostomy tube (56) surrounding the jejunal feeding tube, the gastrostomy tube being adapted to feed and drain the stomach.

8. A device according to claim 7, characterised by a connector (69) for securing the jejunal and gastrostomy feeding tubes (51 and 56) and their respective inlet ends (51B, 56A).

9. A device according to any preceding claim, characterised in that the first abutment is provided by an inflatable balloon structure (16, 32, 65) mounted around the tube and inflatable via an inflation valve (19, 35, 66) mounted near the end of the tube outside the body in use and connected to the balloon structure by a valve line (20, 37, 67) within the tube.

10. A device according to any of claims 1 to 8, characterised in that the first abutment is provided by a flexible plate (82) mounted around the tube and positioned about midway thereof, such that when the tube is inserted through a jejunal placed stoma it will be secured within the stoma by means of the plate (83) which forms a seal with the stoma.

11. A device according to any of claims 1 to 7, characterised in that the first abutment is provided by a mushroom tip (32) which is inserted and removed with a stylet.

## Patentansprüche

1. Katheter für den Magendarmkanal des menschlichen oder tierischen Körpers, umfassend:

einen Schlauch (11, 31, 51, 81), der durch ein Stoma in den Magendarmkanal ragt;

einen Verschluß (22, 45, 60), der den Schlauch verschließt;

ein am Schlauch vorgesehenes erstes Widerlager (16, 32, 65, 82) zur Anlage an der Wand des Magendarmkanals, und

ein entlang dem Schlauch gleitbares zweites Widerlager (17, 33, 40, 70, 83) zur Anlage an der Außenfläche des Körpers um das Stoma herum,

wobei der Schlauch und das Widerlager zur Langzeitimplantation im Körper geeignet sind,

dadurch gekennzeichnet, daß der Reibkontakt zwischen dem Schlauch (11, 31, 51, 81) und dem zweiten Widerlager (17, 33, 40, 70, 83) (a) ausreichend hoch ist, um eine Ziehen des Schlauchs (11, 31, 51, 81) durch das zweite Widerlager (17, 33, 40, 70, 83) durch Peristaltik in den Magen zu verhindern, aber (b) ausreichend gering ist, um eine Bewegung des zweiten Widerlagers (17, 33, 40, 70, 83) nach außen zu gestatten, wenn der peristaltische Druck im Magendarmkanal zu groß wird.

2. Katheter nach Anspruch 1, dadurch gekennzeichnet, daß der Schlauch und das zweite Widerlager aus einem Silikonelastomer medizinischer Güte bestehen.

3. Katheter nach Anspruch 1 oder 2, wobei das zweite Widerlager mehrere Luftlöcher (17A) aufweist, so daß Luft mit der Außenfläche des Körpers in Kontakt gelangen kann.

4. Katheter nach Anspruch 3, wobei das zweite Widerlager Stege oder Rippen (17B, 72) aufweist, so daß Luft zwischen dem zweiten Widerlager und der Außenfläche des Körpers zirkulieren kann.

5. Katheter nach einem der vorhergehenden Ansprüche, dadurch gekennzeichnet, daß der Schlauch eine Ernährungssonde (11) ist, die ein Nahrungseintrittsende (12) und ein perforierties Austrittsende (13) zum Nahrungsaustritt aufweist, und daß der Verschlußstöpsel (22) für den Nahrungseintritt durch ein integrales Band (23) an der Ernährungssonde befestigt ist.

6. Katheter nach einem der vorhergehenden Ansprüche, dadurch gekennzeichnet, daß das zweite Widerlager ein Klemmring (17, 33, 70, 83) ist, durch den der Schlauch im Gleitsitz geführt ist.

7. Katheter nach einem der vorhergehenden Ansprüche, dadurch gekennzeichnet, daß der Schlauch eine jejunale Ernährungssonde (51) und eine die jejunale Ernährungssonde umgebende Gastrostomiesonde (56) aufweist, die zur Zufuhr und zum Ableiten von Nahrung zum bzw. aus dem Magen ausgelegt ist.

8. Katheter nach Anspruch 7, gekennzeichnet durch einen Verbinder (69) zum Befestigen der jejunalen und Gastrostomie-Ernährungssonden (51 und 56) und ihrer jeweiligen Eintrittsenden (51B, 56A).

9. Katheter nach einem der vorhergehenden Ansprüche, dadurch gekennzeichnet, daß das erste Widerlager durch ein den Schlauch umgebend angeordnete aufblasbare Ballonkonstruktion (16, 32, 65) gebildet ist, die über ein Füllventil (19, 35, 66) aufblasbar ist, das bei Verwending nahe dem außerhalb des Körpers befindlichen Schlauchende angeordnet und durch eine Ventilleitung (20, 37, 67) innerhalb des Schlauchs mit der Ballonkonstruktion verbunden ist,

10. Katheter nach einem der Ansprüche 1 bis 8, dadurch gekennzeichnet, daß das erste Widerlager durch eine den Schlauch umgebend angeordnete biegsame Platte (82) gebildet ist, die etwa in der Mitte des Schlauchs so positioniert ist, daß sie bei Einführen des Schlauchs durch ein jejunal angelegtes Stoma in diesem durch die mit dem Stoma eine Dichtung bildende Platte (83) befestigt wird.

11. Katheter nach einem der Ansprüche 1 bis 7, dadurch gekennzeichnet, daß das erste Widerlager durch eine pilzförmiges Ende (32) gebildet ist, das mit einem Führungsstab eingeführt und entfernt wird.

**Revendications**

1. Dispositif à cathéter pour le tube digestif du corps humain ou d'un animal, comprenant:
   un tube (11, 31, 51, 81) destiné à pénétrer par une ouverture dans le tube digestif;
   un obturateur (22, 45, 60) destiné à boucher le tube;
   une première butée (16, 32, 65, 82) prévue sur le tube pour porter contre la paroi du tube digestif, et
   une seconde butée (17, 33, 40, 70, 83) pouvant coulisseur le long du tube pour porter contre la surface extérieure de corps autour de l'ouverture, le tube et la butée convenant à une implantation prolongée sans le corps,
   caractérisé en ce que le contact de frottement entre le tube (11, 31, 51, 81) et la seconde butée (17, 33, 40, 70, 83) est (a) suffisamment élevé pour empêcher la tube (11, 31, 51, 81) d'être tiré à travers la seconde nutée (17, 33, 40, 70, 83) a l'intérieur de l'estomac sous l'effet de mouvements péristaltiques, mais (b) suffisamment faible pour permettre à la seconde butée (17, 33, 40, 70, 83) d'être déplacée vers l'extérieur si la pression péristaltique du tube digestif devient trop grande.

2. Dispositif selon la revendication 1, caractérisé en ce que le tube et la seconde butée sont formés en un élastomère siliconé de qualité médicale.

3. Dispositif selon la revendication 1 ou 2, dans lequel la seconde butée présente plusieurs trous d'évent (17a) pour permettre à l'air d'entrer en contact avec la surface extérieure du corps.

4. Dispositif selon la revendication 3, dans lequel la seconde butée comporte des arêtes ou nervures (17b, 72) pour permettre à l'air de circuler entre la seconde butée et la surface extérieure du corps.

5. Dispositif selon l'une quelconque des revendications précédentes, caractérisé en ce que le tube est un tube (11) d'alimentation ayant une extrémité d'entrée d'alimentation (12) et une extrémité de sortie perforée (13) pour décharger des aliments, et en ce que le bouchon (22) d'obturation pour l'entrée d'alimentation est fixé au tube d'alimentation par une bande (23) réalisée d'une seule pièce avec eux.

6. Dispositif selon l'une quelconque des revendications précédentes, caractérisé en ce que la seconde butée est un anneau de blocage (17, 33, 70, 83) à travers lequel le tube est ajusté à glissement.

7. Dispositif selon l'une quelconque des revendications précédentes, caractérisé en ce que le tube comprend un tube (51) d'alimentation jéjunale et un tube (56) de gastrostomie entourant le tube d'alimentation jéjunale, le tube de gastrostomie étant conçu pour l'alimentation et le drainage de l'estomac.

8. Dispositif selon la revendication 7, caractérisé par un raccord (69) pour fixer les tubes (51 et 56) d'alimentation jéjunale et par gastrostomie et leurs extrémités d'entrée respectives (51B, 56A).

9. Dispositif selon l'une quelconque des revendications précédentes, caractérisé en ce que la première butée est constituée par un structure de ballonnet gonflable (16, 32, 65) montée autour autour du tube et pouvant être gonflée par l'intermédiaire d'une valve (19, 35, 66) de gonflage montée à proximité de l'extrémité du tube extérieure au corps lors de l'utilisation, et reccordée à la structure de ballonnet par une conduite (20, 37, 67) de valve à l'intérieur du tube.

10. Dispositif selon l'une quelconque des revendications 1 à 8, caractérisé en ce que la première butée est constituée par une plaque flexible (82) montée autour du tube et placée sensiblement en son milieu, de manière que, lorsque le tube est inséré à travers une ouverture en position jéjunale, il soit fixé dans l'ouverture au moyen de la plaque (82) qui forme un joint avec l'ouverture.

11. Dispositif selon l'une quelconque des revendications 1 à 7, caractérisé en ce que la première butée est constituée par un embout circulaire (32) qui est inséré et enlevé au moyen d'un stylet.

FIG. I

FIG. 2

FIG.4

FIG.3

STOMACH

STOMA

ABDOMINAL WALL

FIG. 5A

FIG.5B

FIG.6

FIG.7